(19) Europäisches Patentamt
European Patent Office
Office européen des brevets

(11) Publication number : **0 409 999 B1**

# (12) EUROPEAN PATENT SPECIFICATION

(45) Date of publication of patent specification : **15.09.93 Bulletin 93/37**

(51) Int. Cl.$^5$ : **A61K 31/665**

(21) Application number : **90902367.3**

(22) Date of filing : **26.01.90**

(86) International application number : **PCT/JP90/00106**

(87) International publication number : **WO 90/08548 09.08.90 Gazette 90/19**

## (54) LIQUID PREPARATION FOR INTRAOCULAR PERFUSION.

The file contains technical information submitted after the application was filed and not included in this specification

(30) Priority : **02.02.89 JP 125477/89**

(43) Date of publication of application : **30.01.91 Bulletin 91/05**

(45) Publication of the grant of the patent : **15.09.93 Bulletin 93/37**

(84) Designated Contracting States : **AT BE CH DE DK ES FR GB IT LI LU NL SE**

(56) References cited :
**GB-A- 1 431 841**
**JP-A- 5 821 614**
**JP-A-61 246 117**
**JP-A-62 223 117**
**JP-A-62 635 23**
**US-A- 3 812 251**
**US-A- 4 620 979**
**US-A- 4 704 352**
**US-A- 4 725 586**

(73) Proprietor : **Senju Pharmaceutical Co., Ltd.**
**5-8, Hiranomachi 2-chome, Chuo-Ku**
**Osaka-shi, Osaka 541 (JP)**

(72) Inventor : **AWATA, Takashi 203 Tsukaguchi Neo Cooporas**
**22-1, Tsukaguchi-cho 1-chome**
**Amagasaki-shi Hyogo 661 (JP)**
Inventor : **SOGO, Shunji**
**85, Yoogikita 3-chome Yao-shi**
**Osaka 581 (JP)**
Inventor : **MATSUMOTO, Takahiro**
**7-9, Ooakashi-cho 2-chome**
**Akashi-shi Hyogo 673 (JP)**

(74) Representative : **von Kreisler, Alek, Dipl.-Chem. et al**
**Patentanwälte Von Kreisler-Selting-Werner,**
**Postfach 10 22 41, Bahnhofsvorplatz 1**
**D-50462 Köln (DE)**

EP 0 409 999 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### Technical Field

The present invention relates to the use of an intraocular irrigating solution which reduced risks for damage to intraocular tissues, which is used when, in the field of surgical treatment of ophthalmic diseases, the site of operation is inside the eyeball, and more particularly, reduces risks for injury to the intraocular tissues, inter alia the corneal endothelium.

Thus, the present invention relates to the use of L-ascorbic-2-phosphate acid (I)

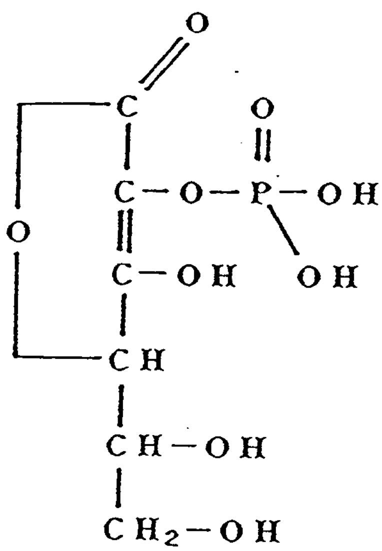

(I)

or a pharmaceutically acceptable salt thereof, for the preparation of an irrigating solution for preventing damages to intraocular tissues.

### Background Art

With the recent rapid advances in surgical techniques in the ophthalmological field, various diseases of the eye in which drug therapy was the only treatment modality or virtually no treatment was available in the past have by now become indications for surgery and the scope of such indications is steadily expanding.

Cataract surgery may be mentioned as the most prevalent of the surgical treatments in the ophthalmological field today and vitrectomy, which is performed for the treatment of e.g. proliferative vitreoretinitis, is another operation that is known well in the field.

Of all the surgical procedures performed in diseases of the eye, cataract surgery and vitrectomy are the most representative, in terms of the number of operated cases, of the operations performed at sites within the eyeball. Since these operations are invariably carried out by incising the cornea or sclera which forms the outermost layer of the bulb and emulsifying or excising and suctioning the damaged tissue with the tip of a surgical instrument introduced into the eyeball, they involve the risk of causing a leakage of aqueous humor or vitreous body from the incised opening during operation and, hence, depressing the intraocular pressure. Furthermore, unless some preventive measure is taken, these operations inflict organic and functional damages on intraocular tissues due to an excessive depression of the intraocular pressure caused by suction of the lesion. For the purpose of precluding these risks, it is common practice to continuously infuse a liquid drug preparation for intraocular irrigation (hereinafter referred to as intraocular irrigating solution) into the vicinity of the site of operation at a constant pressure during operation. This procedure makes up for losses of intrabulbar contents which are caused by leakage of aqueous humor and suction of the damaged tissue, thereby contributing to maintenance of the intraocular pressure within a certain range during operation and, at the same time, washes out the blood and tissue fragments to prevent their interference with the local field of view, thus helping to secure an unobstructed field of operation.

It is known that because the intraocular tissues are constantly contracted with such an intraocular irrigating solution at and near the site of operation during the surgical procedure, it may inflict a temporary or permanent damage on the intraocular tissues, depending on its composition or properties.

Damages to the cornea, inter alia the corneal endothelium, are the most alarming of all the damages to intraocular tissues which may be caused by an intraocular irrigating solution. The corneal endothelium is the innermost (posterior) layer of the three-layer structure of the cornea, a transparent membrane forming,the anterior segment of the eye and consisting of the epithelium, the stroma and the endothelium, and is composed of a single layer of regularly arranged endothelium cells presenting a generally hexagonal pattern. Although the corneal epithelium is a layer as thick as a single cell, it has various functions necessary for the normal physiological condition of the cornea and, hence, plays an important part in maintaining the physiological function of the whole cornea. To mention its major functions, the corneal endothelium controls the water content of the stroma, which is a factor contributing much to corneal transparency, and the selective inter-permeation and transport of substances between the aqueous humor and the corneal stroma. In addition, as the fundamental function necessary for said functions to operate effectively, the corneal endothelium serves as a barrier to the transfer of substances from the cornea to the aqueous humor or vice versa (this function is hereafter referred to as corneal endothelial barrier function). This corneal endothelial barrier function is assured by the intact intercelluar junctional complexes closing up the junctional gaps between endothelial cells, which arrest the free migration of substances from the aqueous humor to the corneal stroma. Thus, the corneal endothelium controls the selective permeation and transfer of water and other substances on the basis of said barrier function, regulating the input-output balance of the whole cornea to maintain the cornea in a wholesome physiological condition and playing an indispensable part in the maintenance of corneal transparency.

Despite the vital part it plays in the maintenance of the normal physiology and transparency of the cornea, the corneal endothelial cells are extremely susceptible to mechanical stimulations associated with surgery or chemical stimulations due to intraocular irrigating solutions as compared with any other intraocular tissues and extraocular body tissues and are, thus, easily lesioned, destroyed and shed off. Furthermore, it is known that, in primates, the endothelial cells in the corneal endothelium once completed have already lost their ability to multiply by cell division. Therefore, if by any cause these cells are destroyed and shed off, the denuded area will little be repaired by proliferation of the surviving endothelial cells but the repairing is mostly effected as the cells in the area adjacent to the denuded area spread into the defected area to cover up the defect. When a desquamation of corneal endothelial cells takes place, the continuity of the corneal endothelium is restored in this manner but the total population of cells constituting the endothelium remains virtually unrestored. When the number of dead and desquamating corneal endothelial cells is large, the cell density per unit area of the corneal endothelium is markedly decreased and as this loss of endothelial cells exceeds a certain limit, the physiological condition of the cornea is no longer maintained well by the endothelium, thus leading to the onset of irreversible corneal opacity and so on.

Since the damage to the endothelial cells is permanent and may lead to a serious disorder such as corneal opacity, the most important requirement which must be fulfilled by an intraocular irrigating solution for surgery is that the solution itself does not inflict an adverse effect on the corneal endothelium. Along the known irrigating solutions designed and prepared to meet the above requirement as much as possible and in clinical use today are Opeguard MA (a trademark of Senju Pharmaceutical Co., Ltd.), BSS (a trademark of Alcon Laboratories Incorporated), and BSS Plus (a trademark of Alcon Laboratories Incorporated).

However, none of the intraocular irrigating solutions available today meet the above-mentioned requirement of preserving the intraocular tissues including corneal endothelial cells fully intact and fundamental research has therefore been undertaken for developing still improved irrigating solutions.

Of the above-mentioned various functions of the corneal endothelium which are of paramount importance for maintenance of the physiological function of the whole cornea, i.e. the function of controlling the water content of the corneal stroma, the function of assuring a selective permeation and transport of various substances from the aqueous humor to the corneal stroma and vice versa, and the corneal endothelial barrier function, the last-mentioned corneal endothelial barrier function is easily and rapidly impaired even by the slightest damage inflicted on the endothelial cells and, therefore, is of value as a sensitive indicator of corneal endothelial impairment. Moreover, its hydrofunction can be experimentally titrated with high accuracy. For these reasons, the study of corneal endothelial barrier function has recently been attracting attention as a useful approach to elucidation of the influence of intraocular irrigating solutions on the corneal endothelium. An exemplary relevant technique comprises removing the epithelium from the cornea, exposing the de-epithelialized side of the cornea to a solution of 5(6)-carboxyfluorescein (hereinafter referred to briefly as CF), which is a fluorescent substance, determining the amount of CF translocated to the anterior chamber side at timed intervals (Araie: Arch. Ophthalmol. 104:435, 1986). It is known that when the corneal endothelial barrier function is wholesome, the fluorescent substance CF virtually does not pass through the corneal endothelium, i.e. CF does not migrate from the epithelial side to the anterior chamber side or vice versa. Therefore, when the epithelial side of the cornea is exposed.to a CF solution and the amount of CF migration from the endothelial side is determined, the degree of gaps created at the junctional complexes of endothelial cells, that is to say the degree of impairment of corneal endothelial barrier function, can be quantitated and, hence the degree of injury to the corneal epithelial cells can be ascertained.

On the other hand, it is known that as the physiological function of the corneal endothelium is impaired, there occurs an abnormal penetration of water into the corneal stroma to swell the stroma and, hence, increase the thickness of the cornea as a whole. By irrigating the endothelial side of the cornea isolated from an animal eye with an intraocular irrigating solution or by irrigating the anterior chamber of an animal eye in situ and measuring the change induced in the thickness of the cornea, the effect of the intraocular irrigating solution can be directly determined (H. F. Edelhauser et al., Arch. Ophthalmol., 93:648 (1975) and Teruo Yajima et al. Jpn. Rev. Clin. Ophthalmol., 75:1189 (1981).

## Disclosure of Invention

The inventors of the present invention endeavored to discover a substance which would eliminate or markedly reduce the tissue toxicity of the intraocular irrigating solution and, using the method for assessing the corneal endothelial barrier function (described in Experiment 1) employing a cultured endothelial cell monolayer which was established by improving the above-mentioned method of Shinge et al. and the above-mentioned in vitro method for determining the corneal thickness, studied the effects of various new intraocular irrigating solutions prepared by adding various substances to the known basic intraocular irrigating solution. The experiments using these preparations for evaluation of their effects on the cornea revealed surprisingly that a certain phosphoric ester of L-ascorbic acid inhibits the onset of corneal damage. The present invention is the result of the above finding and subsequent research.

Thus, the present invention relates to an intraocular irrigating solution, which is characterized by containing said known substance (I)

[Blank]

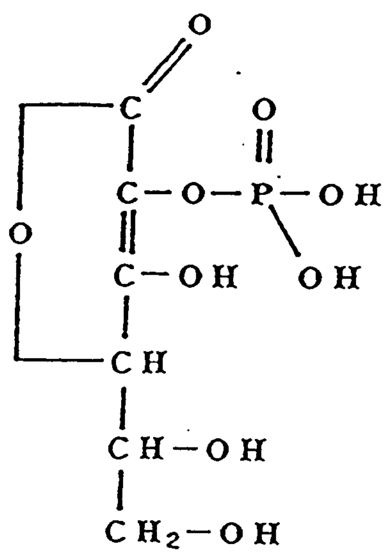

(I)

or a pharmaceutically acceptable salt thereof. Substance (I) and its various salts such as the sodium, potassium, calcium and magnesium salts are known to be useful stabilized ascorbic acid preparations as disclosed, for example, in Japanese Patent Application KOKAI No. 62-285759. Other known uses for substance (I) and salts thereof include antioxidants as disclosed, for example, in Japanese Patent Application KOKAI No. 63-95287, an agent for preventing dysplasia of the eggshell as disclosed, for example, in Japanese Patent Application KOKAI No. 62-198615, an agent for preventing body shape abnormally in fish as disclosed, for example, in Japanese Patent Application KOKAI NO. 62-175142, as a skin depigmenting agent as disclosed, for example, in Japanese Patent Application KOKAI NO. 62-96405, a stabilizer for preserved erythrocytes as described, for example, in Japanese Patent Application KOKAI No. 62-63616, a mouth deodorant as disclosed in Japanese Patent Application KOKAI No. 62-96408. However, as mentioned hereinbefore, none of the published literature suggest the effectiveness of substance (I) and its pharmaceutically acceptable salts against damage to corneal endothelial cells. Thus, this effect was discovered for the first time in the course of development of the present invention.

Examples of said pharmaceutically acceptable salt of substance (I) which can be used in the intraocular irrigating solution of the invention are alkali metal salts such as sodium salt, potassium salt, etc., alkaline earth metal salts such as calcium salt, magnesium salt and so on, and these salts can be used singly or in combination.

In formulating the intraocular irrigating solution of the present invention, substance (I) can be advantageously used in combination with sodium chloride and potassium chloride which are commonly used in the manufacture of intraocular irrigating solutions. The concentration of sodium chloride is preferably in the range of 80 mmol/l to 125 mmol/l and the concentration of potassium chloride is preferably in the range of 4 mmol/l to 10 mmol/l. Among such other ingredients which are commonly used in the manufacture of intraocular irrigating solutions are various electrolytes such as calcium chloride, magnesium chloride, magnesium sulfite, sodium acetate, sodium phosphate, potassium phosphate, sodium bicarbonate, etc., carbohydrates such as glucose etc., peptides such as glutathione, glutathione disulfide, etc., and nucleosides such as adenosine and so on.

The preferred amount of substance (I) or said pharmaceutically acceptable salt thereof in the intraocular irrigating solution of the present invention is in the range of 0.25 mmol/l - 20.0 mmol/l, and more preferably in the range of 0.5 mmol - 10.0 mmol/l and, for still better results, in the range of 1.0 mmol/l - 5.0 mmol/l.

The preferred pH of the intraocular irrigating solution of the invention is in the range of 7.0 to 7.5. Its osmotic pressure is preferably adjusted to the range of 260 mOsm - 310 mOsm and more preferably to the range 275 mOsm - 305 mOsm.

The effects of the intraocular irrigating solution of the invention is shown below on the protection of corneal endothelial barrier function and the protection against corneal swelling.

Experiment 1: Effect on corneal endothelial barrier function

Method

[Preparation of corneal endothelial cells]

The eyeballs of male white rabbits were enucleated and in a culture medium prepared by adding 10 % fetal calf serum to Dulbecco's Modified MEM (trademark: Dulbecco's Modified Eagle Medium "Nissui" 2, Nissui Pharmaceutical), (hereinafter referred to as DMEM), the cornea was cut out along the corneosclerai limbus with a pair of scissors. From each of the isolated corneal specimens (8 eyes), the endothelium with Descemet's membrane was peeled off, shorn into small pieces, and seeded with about 5 ml of DMEM in a 75 $cm^2$ culture flask (trademark: Nunclon Tissue Culture Flask 147589; manufactured by Nunc). After adhesion of the cells to the bottom of the flask, about 20 ml of DMEM was added and the cells were cultured for about 7 days until the growth on the internal wall of the flask became confluent. During this period, the medium was replaced with fresh one every 3 days. After completion of culture, the cells were digested with 5 ml of EDTA-trypsin (a phosphate buffer containing 0.01 % of EDTA and 0.125 % of trypsin and not containing calcium or magnesium ion) and, then, mixed with a sufficient quantity of DMEM to terminate the digestion. The digest was centrifuged at 1000 rpm for 2 minutes and the cells were then collected. The harvested cells were re-suspended in DMEM and filtered through a 200μ m nylon filter to remove Descemet's membrane, leaving an exclusive suspension of cells. This cell suspension was diluted with DMEM to make 20 ml and a 300-μ l portion of the dilution was seeded on the upper surface of the permeable collagen membrane set in position across the bottom opening of a plastic hollow cylinder (10 mm in inside diameter X 6 mm in height) having 2 mm-high shanks in four circumferential positions (trademark: Cellgen, permeable membrane CM-24; manufactured by K.K. Koken; hereinafter referred to as "Permeation Cell".). In this condition, the cells were cultured at 37°C for 2 days.

[Culture in test solutions]

After culture, the Permeation Cell was set in a 10 cm (dia.) dish containing a test solution and the endothelial cells on the permeable collagen membrane were gently washed with the test solution. The Permeation Cell was then transferred to a separate 10 cm (dia.) at 37°C under 5 % $CO_2$-95% air for 4 hours.

[Test solutions]

The test solutions used were the intraocular irrigating solutions described in Examples 1 and 2, respectively, and the intraocular irrigating solution of the following composition (hereinafter referred to as the standard solution) which was identical with the above-mentioned test solutions in composition, pH and osmotic pressure

except that 1.2 mmol/l of magnesium sulfate was contained in lieu of substance (I).

<Composition of standard solution (Unit: mmol/l)>

| | |
|---|---|
| Magnesium sulfate | 1.2 |
| Calcium chloride | 1.2 |
| Sodium chloride | 112.9 |
| Potassium Chloride | 4.8 |
| Sodium acetate | 4.4 |
| Sodium citrate | 3.4 |
| Glucose | 8.3 |
| Sodium bicarbonate | 25.0 |

pH 7.3, osmotic pressure 290 mOsm

[Determination of the amount of CF penetration]

CF was dissolved in each test solution at a final concentration of 0.1 % and the solution was filtered through a 0.45-μ m filter to give a 0.025 % solution of CF (hereinafter referred to as CF solution). The wells of a 12-well culture plate molded one-piece with 12 cylindrical wells each measuring 23 mm in inside diameter and 13 mm in depth (Clusters Dish 3512; manufactured by Costar) were filled with 2 ml portions of the test solutions. The Permeation Cell incubated with the corresponding test solution was set in an inverted position on a sheet of filter paper to blot off the solution from within the Cell and, then, set in an upright position in the culture plate well containing the corresponding test solution. Then, 300 μ l of the CF solution corresponding test solution. Then, 300 μ l of the CF solution corresponding to the particular test solution was put in the cell and cultured at 37°C under 5 % $CO_2$-95 % air. After the beginning of culture, the test solution outside of the Permeation Cell was sampled at 20-minute intervals in aliquots of 200 μ l for use as samples for determination of CF. After this sampling procedure, the culture wells were immediately refilled with 200 μ l portions of the respective test solutions. Each of the samples taken was diluted 10-fold and the intensity of fluorescence of the dilution was measured with a spectrofluorophotometer (Model F3000, Hitachi) at an excitation wavelength of 497 nm and an emission wavelength of 543 nm. The CF concentration was then calculated.

Results

As shown in Table 1, the amount of CF penetrating through the corneal endothelial cell monolayer on the permeable collagen membrane of the Permeation Cell during incubation decreased with an increasing concentration of substance (I) in the test solution. This result indicates that substance (I) inhibits the permeation of CF through the cultured corneal endothelial cell monolayer, suggesting strongly that substance (I) protects the corneal endothelial barrier function.

Table 1 The amount of permeation of CF

| Test solution (Number of cases) | The amount of permeation of CF (μg) Standard solution (6) | Example 1 (7) | Example 2 (7) |
|---|---|---|---|
| 0 Minute | 0 | 0 | 0 |
| 20 Minutes | 3.54 ± 0.58 | 3.09 ± 0.33 | 2.95 ± 0.52 |
| 40 Minutes | 7.41 ± 0.98 | 6.78 ± 0.66 | 6.04 ± 1.06 |
| 60 Minutes | 11.01 ± 1.50 | 10.05 ± 0.88 | 8.84 ± 1.37 |

(Mean ± S.D.)

Experiment 2: Effect on the swelling of cultured cornea Method

Method

The eyeballs of adult rabbits were enucleated together with the conjuctivae and eyelids and according to the method of Dikstein et al. (Dikstein S. et al.: J. Physiol., 221:29, 1972), each specimen was fixed in a corneal irrigation apparatus. Then, using an automatic infusion apparatus (KN 204; manufactured by Natsume Seisakusho, Ltd.), the endothelial side of the cornea was irrigated with one of the under-mentioned test solutions under the following conditions. The irrigating solution temperature: 37°C, rate of irrigation: 0.027 ml/min., irrigating pressure: 15 mmHg. To prevent drying of the epithelial side of the cornea, the epithelial side was protected with silicone oil (Shin-Etsu Chemical Industries, Ltd.). The thickness of the cornea was measured at 1-hour intervals for 4 hours using a specular microscope (trademark: Kohnan Clinical Specular Microscope 400VZ, Kohnan Camera, Ltd.) with a pachometer attachment.

[Test solutions]

The test solutions used were the intraocular irrigating solution described in Example 1 and the standard solution.

Results

It is apparent from the diagram shown on the accompanying drawing that, compared with the cornea irrigated with the control standard solution, the cornea irrigated with the intraocular irrigating solution containing substance (I) (3/2 magnesium salt) according to Example 1 markedly inhibited the increase in cultured corneal thickness which is an indicator of abnormal swelling of the corneal stroma. Since the control standard solution contained approximately the same amount of magnesium ion as the intraocular irrigating solution of Example 1, the observed effect of the latter intraocular irrigating solution was considered to be the effect intrinsic to the substance (I).

**Brief Description of the Drawings**

The accompanying drawing shows the increases in corneal thickness caused by irrigation of the rabbit corneas. The abscissa represents the time after the beginning of irrigation and the ordinate represents change (increments) in corneal thickness.

**Best Mode for Carrying Out the Invention**

Example 1:

The following ingredients in the indicated amounts were successively dissolved in distilled water and the solution was adjusted to the indicated pH with hydrochloric acid and adjusted to the indicated pH with hydrochloric acid and aseptically filtered to give an intraocular irrigating solution.

[Composition (Unit: mmol/l)

| | |
|---|---|
| Calcium chloride | 1.2 |
| Sodium Chloride | 112.9 |
| Potassium chloride | 4.8 |
| Sodium acetate | 4.4 |
| Sodium citrate | 3.4 |
| Glucose | 8.3 |
| Substance (I) (3/2 magnesium salt) | 1.0 |
| Sodium bicarbonate | 25.0 |

pH 7.3; osmotic pressure 290 mOsm

Example 2;

The following ingredients in the indicated amounts were successively dissolved in distilled water and the solution was adjusted to the indicated pH with hydrochloric acid and aseptically filtered to give an intraocular irrigating solution.

[Composition (Unit: mmol/l)

| | |
|---|---|
| Calcium chloride | 1.2 |
| Sodium chloride | 112.9 |
| Potassium chloride | 4.8 |
| Sodium acetate | 4.4 |
| Sodium citrate | 3.4 |
| Glucose | 8.3 |
| Substance (I) (3/2 magnesium salt) | 3.0 |
| Sodium bicarbonate | 25.0 |

pH 7.3; osmotic pressure 290 mOsm

**Industrial Applicability**

The present invention provides a highly safe intraocular irrigating solution which contains substance (I) or a pharmaceutically acceptable salt thereof. As described hereinbefore in Experiments, the intraocular irrigating solution protects the above-described barrier function of the corneal endothelium and inhibits abnormal swelling of the cornea which is suspected to occur due to hypofunction of the corneal endothelium. Therefore, the use of the intraocular irrigating solution of the present invention results in prevention of the damage to the corneal endothelium and helps maintain the whole cornea in normal physiological condition, thus precluding chances for corneal opacity and other intraocular tissues disorders due to indispensable intraocular irrigation for intraocular surgery and, hence, assuring an enhanced safety of surgery.

## Claims

1. Use of L-ascorbic -2-phosphate acid (I)

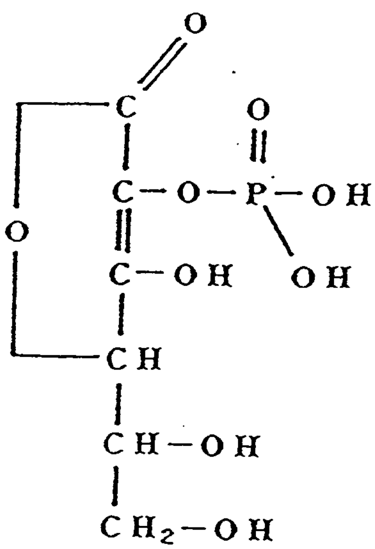

( I )

   or a pharmaceutically acceptable salt thereof for the preparation of an irrigating solution for preventing damages to intraocular tissues.

2. Use of an intraocular irrigating solution of Claim 1 which contains said substance (I) or a pharmaceutically acceptable salt thereof in a proportion of 0.25 mmol/l to 20.0 mmol/l.

3. Use of an intraocular irrigating solution of Claim 2, in which its pH is in the range of 7.0 to 7.5 and its osmotic pressure is in the range of 260 mOsm to 310 mOsm.

## Patentansprüche

1. Verwendung der L-Ascorbin-2-phosphat-säure (I)

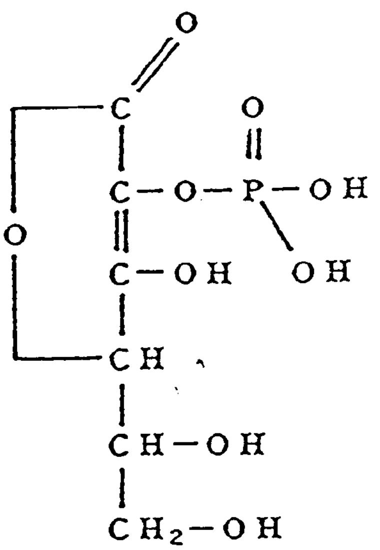

( I )

oder eines pharmazeutisch annehmbaren Salzes derselben zur Herstellung einer Ausspül-Lösung zur Verhinderung von Schädigungen an intraokulären Geweben.

2. Verwendung einer intraokulären Ausspül-Lösung nach Anspruch 1, die die genannte Substanz (I) oder ein pharmazeutisch annehmbares Salz derselben in einem Anteil von 0,25 mmol/l bis 20,0 mmol/l enthält.

3. Verwendung einer intraokulären Ausspül-Lösung nach Anspruch 2, deren pH-Wert im Bereich von 7,0 bis 7,5 liegt und deren osmotischer Druck im Bereich von 260 mosm bis 310 mosm liegt.

## Revendications

1. Utilisation de l'acide L-ascorbique-2-phosphorique (I)

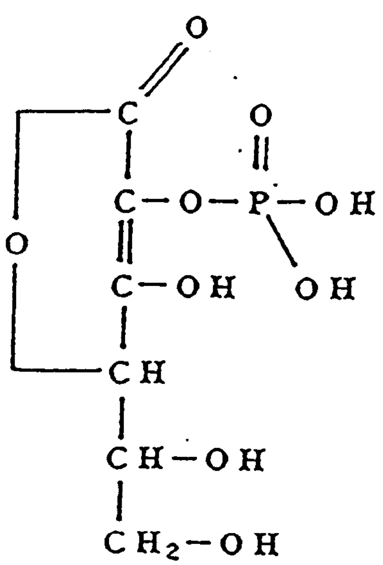

( I )

ou d'un de ses sels pharmaceutiquement acceptables, pour préparer une solution d'irrigation destinée à prévenir les dommages aux tissus intraoculaires.

2. Utilisation d'une solution pour irrigation intraoculaire selon la revendication 1, qui contient ladite substance (I) ou l'un de ses sels pharmaceutiquement acceptables en une quantité de 0,25 à 20,0 mmol/l.

3. Utilisation d'une solution pour irrigation intraoculaire selon la revendication 2, dont le pH est de 7,0 à 7,5 et dont la pression osmotique est de 260 à 310 mOsm.

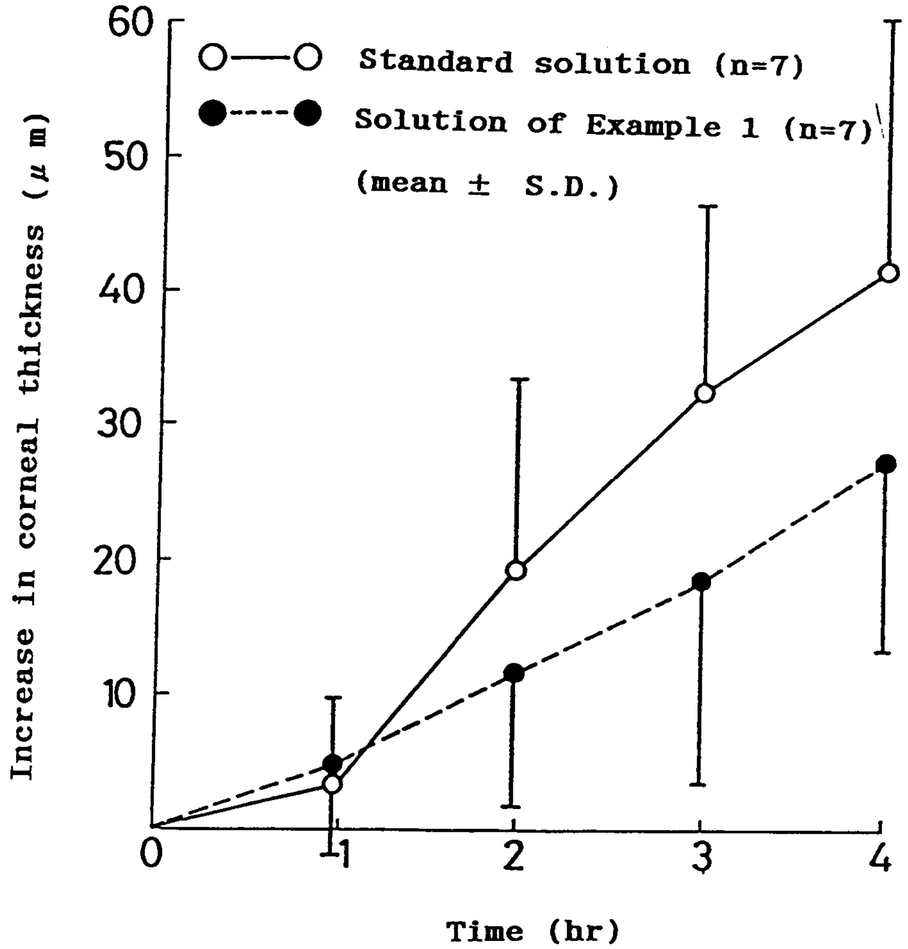

60
50
40
30
20
10
0
1
2
3
4
Standard solution (n=7)
Solution of Example 1 (n=7)
(mean ± S.D.)
Increase in corneal thickness (μ m)
Time (hr)